# EUROPÄISCHE PATENTSCHRIFT

(11) **EP 1 417 192 B1**
(45) Veröffentlichungstag und Bekanntmachung des Hinweises auf die Patenterteilung: **20.02.2008**
(21) Anmeldenummer: 02791483.7
(22) Anmeldetag: 30.07.2002
(51) Int. Cl.: C07D 301/12, C07D 301/32

(54) **VERFAHREN ZUR HERSTELLUNG VON PROPYLENOXID**
METHOD FOR THE PRODUCTION OF PROPYLENE OXIDE
PROCEDE DE FABRICATION D'OXYDE DE PROPYLENE

(30) Priorität: 01.08.2001 DE 10137543
(43) Veröffentlichungstag der Anmeldung: 12.05.2004
(73) Patentinhaber: BASF Aktiengesellschaft, 67056 Ludwigshafen (DE)
(72) Erfinder: TELES, Joaquim, Henrique, 67166 Otterstadt (DE); REHFINGER, Alwin, 67112 Mutterstadt (DE); BERG, Anne, 2170 Merksem (BE); RUDOLF, Peter, 68526 Ladenburg (DE); RIEBER, Norbert, 68259 Mannheim (DE); BASSLER, Peter, 68519 Viernheim (DE)
(74) Vertreter: Isenbruck, Günter
(86) Internationale Anmeldenummer: PCT/EP2002/008487
(87) Internationale Veröffentlichungsnummer: WO 2003/011845

(56) Entgegenhaltungen:
- EP-A- 0 719 768
- DE-A- 1 668 666
- DE-A- 19 946 134
- US-A- 5 599 955
- US-A- 5 773 634
- US-A- 5 849 937
- US-A- 6 160 137

## Beschreibung

Die vorliegende Erfindung betrifft ein Verfahren, in welchem Propylenoxid aus Wasserstoffperoxid und Propen hergestellt wird, wobei ein Gemisch anfällt, welches nicht-umgesetztes Propen und Sauerstoff aufweist, das anschließend verwertet wird.

Bei vielen Propylenoxid-Herstellungsverfahren mit Propen als Edukt wird angestrebt, das im Verfahren nicht-umgesetzte Propen möglichst vollständig aus dem Produktaustrag zurückzugewinnen, gegebenenfalls zu reinigen und erneut als Edukt in das Verfahren zurückzuführen. In einer möglichen Ausführungsform wird hierbei dieser Produktaustrag einer Destillation unterworfen, bei welcher das nicht-umgesetzte Propen zusammen mit Verbindungen mit einem niedrigeren Siedepunkt als Propen aus dem Produktaustrag entfernt wird. Im Anschluss daran wird aus dieser Leichtsiedefraktion das nicht-umgesetzte Propen abgetrennt, gegebenenfalls aufbereitet und wieder in das Verfahren zurückgeführt. Derartige Verfahren sind u.a. in der DE 10001401.1 beschrieben.

Bei der Abtrennung von Propen stellt sich jedoch häufig das Problem, dass neben Propen noch Sauerstoff in einer Konzentration vorliegen kann, welche zur Bildung zündfähiger Gemische führt. Daher stellt die Abtrennung des Propens ein ernstes Sicherheitsrisiko dar.

Zur sicheren Aufarbeitung bzw. Abtrennung des Propens, ist es daher notwendig, die Entstehung eines zündfähigen Gemisches aus Propen und Sauerstoff zu vermeiden. Zu diesem Zweck schlägt beispielsweise die EP-B 0 719 768 vor, die destillative Abtrennung des nicht-umgesetzten Propens aus der Leichtsiedefraktion in einer sogenannten Absorptionszone durch ein geeignetes Absorptionsmittel vorzunehmen, sowie durch die zusätzliche Zugabe eines Inertgases in diese Absorptionszone, den Sauerstoff bis zu einer Konzentration zu verdünnen, bei welcher das Gemisch nicht mehr zündfähig ist.

US 5,773,634 betrifft ein Verfahren zur Umsetzung von Sauerstoff und Isobutan. Dabei betrifft das offenbarte Verfahren die Wiedergewinnung von dampfförmige Kohlenwasserstoffen wie Propylen von gasförmigen Produkten aus dem Reaktor. Dabei wird offenbart, dass das Abgas auch als Brennstoff genutzt werden kann.

US 5,849,937 betrifft ein Verfahren zur Epoxidierung in Gegenwart eines heterogenen Titan-Silicalit-Katalysators. Dabei wird gemäß US 5,849,937 nach Durchlaufen des letzten Reaktors der Reaktorkaskade der Zustrom fraktioniert oder anderweitig behandelt, wobei nicht umgesetztes Olefin zurückgeführt werden kann.

US 6,160,137 betrifft ein Verfahren zur Herstellung von Propylenoxid aus einem Hydroperoxid und Propylen, wobei nicht umgesetztes Propylen nach der Aufarbeitung in das Epoxidationsverfahren zurückgeführt werden kann.

DE 1 668 666 A1 betrifft ein kontinuierliches Verfahren zur Herstellung von Ethylenoxid. Dabei wird offenbart, dass das ausgetragene Gas, das Sauerstoff und nicht umgesetztes Ethylen enthalten kann, beispielsweise dazu genutzt wird, Druckluft zu erzeugen, die wiederum zur Direktoxidation eingesetzt werden kann.

In einer weiteren Patentanmeldung DE 10001401.1, welche ebenfalls ein Verfahren zur Herstellung von Propylenoxid betriff, wird durch die im Folgenden aufgeführte Verfahrensweise vermieden, dass es bei der Abtrennung von nicht-umgesetzten Propen aus einem Propen und Sauerstoff umfassenden Gemisch zur Entstehung eines zündfähigen Gemisches kommt. Im Rahmen dieser Patentanmeldung werden zunächst Propen und ein Hydroperoxid in einem Lösungsmittel in Anwesenheit eines Titansilikalit-Katalysators zu Propylenoxid unter Erhalt eines Gemisches umgesetzt, welches neben weiteren Komponenten auch nicht-umgesetztes Propen und Sauerstoff aufweist. Aus diesem Gemisch wird durch einen katalytischen Prozess Sauerstoff unter Erhalt eines weiteren Gemisches, umfassend Propen, entfernt, wobei aus diesem weiteren Gemisch Propen destillativ abgetrennt und als Edukt in das Verfahren rückgeführt wird.

Eine weitere Möglichkeit zur sicheren Aufarbeitung wird in einer weiteren Patentanmeldung der Anmelderin beschrieben. Diese Anmeldung betrifft ein Verfahren zur Herstellung von Propylenoxid, wobei ein Gemisch welches nicht-umgesetztes Propen und Sauerstoff aufweist derart von dem Produktaustrag abgetrennt wird, dass es nicht zündfähig ist. Die Nicht-Zündfähigkeit dieses Gemisches wird dadurch erreicht, dass die Konzentration von Sauerstoff im Gemisch weniger als 12 Vol.-% beträgt.

Demgemäss erfordert die sichere Aufarbeitung der das nicht-umgesetzte Propen enthaltenden Leichtsiedefraktion einen erhöhten apparativen sowie einen unmittelbar damit verbundenen erhöhten Energie-Aufwand. Dadurch gestaltet sich das Gesamtverfahren, d.h. die Herstellung von Propylenoxid verbunden mit der Rückgewinnung und Rückführung nicht-umgesetzten Propens, häufig energieineffizient. Folglich erscheint die Rückgewinnung von nicht-umgesetztem Propen für den Zweck der Rückführung in das Verfahren in der wirtschaftlichen Gesamtbetrachtung des Verfahrens häufig als unrentabel.

Selbstverständlich besteht jedoch gerade in der heutigen Zeit, nicht nur aufgrund des Gedankens der Ressourcenschonung, das Bedürfnis, jedes teilweise nicht-umgesetzte Edukt oder nicht unmittelbar im weiteren Verfahren nutzbare Zwischenprodukt auf wirtschaftlich sinnvolle Weise aufzuarbeiten und somit das Verfahren als Ganzes ökonomischer und damit letztlich auch wettbewerbstauglicher zu gestalten.

Somit lag der vorliegenden Erfindung die Aufgabe zugrunde ein gegenüber dem Stand der Technik effizienteres Verfahren zur Herstellung von Propylenoxid bereitzustellen.

Demgemäss betrifft die vorliegende Erfindung ein Verfahren zur Herstellung von Propylenoxid, welches wenigstens die folgenden Schritte aufweist:
(a) Propen wird mit Wasserstoffperoxid in einem Lösungsmittel in Anwesenheit eines Zeolith-Katalysators unter Erhalt eines Gemisches (G0) umgesetzt, wobei das Gemisch (G0) mindestens Propylenoxid, Lösungsmittel, nicht-umgesetztes Propen, nicht-umgesetztes Wasserstoffperoxid und Sauerstoff aufweist,
(b) das Propylenoxid wird aus Gemisch (G0) derart abgetrennt, dass ein Gemisch (G1) erhalten wird, welches nicht-umgesetztes Propen und Sauerstoff enthält, wobei
(c) das Gemisch (G1) verwertet wird, wobei im Rahmen dieser Verwertung das im Gemisch (G1) enthaltene Propen nicht in die Umsetzung gemäß (a) rückgeführt wird und wobei das Gemisch (G1) zur Energiegewinnung eingesetzt wird, wobei das Gemisch (G1)
   (aa) wenigstens einer weiteren Aufarbeitungsvorrichtung zugeführt wird,
   (bb) in dieser verbrannt wird und
   (cc) die gemäß (bb) entstehende Verbrennungswärme zur Wasserdampfgewinnung eingesetzt wird, wobei der Wasserdampf als Energieträger zum Betreiben von Destillationskolonnen in dem Verfahren zur Herstellung von Propylenoxid eingesetzt wird.

Schritt (a) des erfindungsgemäßen Verfahrens kann nach allen dem Fachmann für diese Umsetzung bekannten Verfahrensführungen, insbesondere nach den in den Patentanmeldungen DE 19835907.1, DE 19936547.4 , DE 10015246.5 sowie DE 10032885.7 durchgeführt werden.

Bevorzugt wird die Umsetzung von Propen mit Wasserstoffperoxid in einem Lösungsmittel in Anwesenheit eines geeigneten Katalysators zu einem Gemisch (G0) in wenigstens einem Rohrbündelreakor durchgeführt.

Im Rahmen der vorliegenden Erfindung können grundsätzlich alle dem Fachmann geeignet erscheinenden Lösungsmittel eingesetzt werden. Beispielsweise können als Lösungsmittel
- Wasser,
- Alkohole, bevorzugt niedere Alkohole, weiter bevorzugt Alkohole mit weniger als 6 Kohlenstoffatomen wie beispielsweise Methanol, Ethanol, Propanole, Butanole, Pentanole,
- Diole oder Polyole, bevorzugt solche mit weniger als 6 Kohlenstoffatomen,
- Ether wie beispielsweise Diethylether, Tetrahydrofuran, Dioxan, 1,2-Diethoxyethan, 2-Methoxyethanol,
- Ester wie beispielsweise Methylacetat oder Butyrolacton,
- Amide wie beispielsweise Dimethylformamid, Dimethylacetamid, N-Methylpyrrolidon,
- Ketone wie beispielsweise Aceton,
- Nitrile wie beispielsweise Acetonitril
- oder Gemische aus zwei oder mehr der vorgenannten Verbindungen eingesetzt werden.

Bevorzugt wird im Rahmen der Erfindung als Lösungsmittel Methanol eingesetzt.

Als Katalysatoren in Schritt (a) werden im Rahmen der vorliegenden Erfindung Zeolith-Katalysatoren eingesetzt werden.

Bevorzugt werden Zeolithe eingesetzt, welche Eisen-, Titan-, Vanadium-, Chrom-, Niob- oder Zirkonium-haltig sind.

Dabei sind im einzelnen Titan-, Germanium-, Tellur-, Vanadium-, Chrom-, Niob-, Zirkonium-haltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen zu nennen. Denkbar sind für den Einsatz im erfindungsgemäßen Verfahren weiterhin titanhaltige Zeolithe mit der Struktur des ITQ-4, SSZ-24, TTM-1, UTD-1, CIT-1 oder CIT-5. Als weitere titanhaltige Zeolithe sind solche mit der Struktur des ZSM-48 oder ZSM-12 zu nennen.

Bevorzugt werden im Rahmen der vorliegenden Erfindung Ti-Zeolithe mit MFI-, MEL- oder MFI/MEL-Mischstruktur eingesetzt. Als weiterhin bevorzugt sind im einzelnen die Tienthaltenden Zeolith-Katalysatoren, die im allgemeinen als "TS-1", "TS-2", "TS-3" bezeichnet werden, sowie Ti-Zeolithe mit einer zu β-Zeolith isomorphen Gerüststruktur zu nennen.

Demgemäss wird in dem in Rede stehenden erfindungsgemäßen Verfahren, wie oben beschrieben, besonders bevorzugt als Zeolith-Katalysator ein Titansilikalit-Katalysator, insbesondere ein Titansilikalit-Katalysator der Struktur TS-1 eingesetzt.

Weitere Details bezüglich der einsetzbaren Zeolithe sind der DE 10010139.2 zu entnehmen.

Das aus der Umsetzung in Schritt (a) hervorgehende Gemisch (G0) weist im wesentlichen folgende Komponenten auf: Propylenoxid als gewünschtes Verfahrensprodukt, Lösungsmittel, Wasser, nicht-umgesetztes Hydroperoxid, nicht-umgesetztes Propen und Sauerstoff.

Im Rahmen der vorliegenden Erfindung ist es selbstverständlich auch möglich, Propen einzusetzen, welches bis zu 10 Gew.-% Kohlenwasserstoffe, die verschieden von Propen sind, aufweist.

Beispielsweise kann das eingesetzte Propen bis zu 10 Gew.-% Propan, Ethan, Ethylen, Butan oder Butene einzeln oder als Gemisch aus zwei oder mehr davon aufweisen.

Aus dem aus Schritt (a) des erfindungsgemäßen Verfahrens resultierenden Gemisch (G0) wird in einem weiteren Schritt (b) Propylenoxid derart abgetrennt, dass ein Gemisch (G1) erhalten wird, welches wenigstens nicht-umgesetztes Propen und Sauerstoff aufweist.

Die Abtrennung von Propylenoxid aus dem Gemisch (G0) in Schritt (b) des erfindungsgemäßen Verfahrens kann nach jeder, dem Fachmann bekannten Verfahrensführung für eine derartige Abtrennung durchgeführt werden.

Somit können zwischen Schritt (a) und Schritt (b) des erfindungsgemäßen Verfahrens selbstverständlich weitere, dem Fachmann bekannte und im Zusammenhang mit einem Verfahren zur Herstellung von Propylenoxid geeignet erscheinende Zwischenschritte eingefügt werden.

Das aus Schritt (b) hervorgehende Gemisch wird sodann in einem weiteren Schritt des erfindungsgemäßen Verfahrens, Schritt (c) verwertet.

Erfindungsgemäß wird das Gemisch (G1) in Schritt (c) zur Energiegewinnung eingesetzt.

Im Rahmen der Erfindung wird das gasförmige Gemisch (G1), welches von Gemisch (G0) in der oben beschriebenen Weise abgetrennt wurde, zu diesem Zweck zunächst wenigstens einer weiteren Aufarbeitungsvorrichtung zugeführt. In dieser wird das Gemisch (G1) vorzugsweise mit weiterem Sauerstoff versetzt und anschließend verbrannt. Die dabei freiwerdende Wärmenergie kann beispielsweise in wirtschaftlich verwertbare Energieform umgewandelt werden.

Die im Schritt (c) freigesetzte Energie wird zur Erzeugung von Wasserdampf eingesetzt.

Somit wird im Rahmen der Erfindung die in Schritt (c) anfallende Verbrennungswärme zur Erwärmung eines fluiden Mediums zum Zwecke der Dampfgewinnung eingesetzt (Dampfgenerierung). Dieser so generierte Dampf kann auf verschiedenste Weise im oben genannten Verfahren nutzbringend eingesetzt werden.

Eine wirtschaftlich sinnvolle Nutzung des Dampfes ist beispielsweise die direkte Erwärmung von im Verfahren eingesetzten Vorrichtungen. Weiterhin kann der Dampf in weitere, wirtschaftlich innerhalb des Verfahrens nutzbare Energieformen umgewandelt werden, beispielsweise über dazu dem Fachmann bekannte Transformatoren in mechanische oder elektrische Energie.

Vorteilhafterweise wird die dabei gewonnene mechanische oder elektrische Energie im erfindungsgemäßen Verfahren zum Betrieb der innerhalb des Verfahrens eingesetzten Vorrichtungen verwendet, so dass damit eine energieeffiziente und damit umweltfreundliche Verfahrensführung erreicht wird.

Demgemäss wird der erzeugte Wasserdampf als Energieträger zum Betreiben von Destillationskolonnen im erfindungsgemäßen Verfahren eingesetzt.

## Patentansprüche

1. Verfahren zur Herstellung von Propylenoxid, welches wenigstens die folgenden Schritte aufweist:
(a) Propen wird mit Wasserstoffperoxid in einem Lösungsmittel in Anwesenheit eines Zeolith-Katalysators unter Erhalt eines Gemisches (G0) umgesetzt, wobei das Gemisch (G0) mindestens Propylenoxid, Lösungsmittel, nicht-umgesetztes Propen, nicht-umgesetztes Wasserstoffperoxid und Sauerstoff aufweist,
(b) das Propylenoxid wird aus Gemisch (G0) derart abgetrennt, dass ein Gemisch (G1) erhalten wird, welches nicht-umgesetztes Propen und Sauerstoff enthält, wobei
(c) das Gemisch (G1) verwertet wird, wobei im Rahmen dieser Verwertung das im Gemisch (G1) enthaltene Propen nicht in die Umsetzung gemäß (a) rückgeführt wird und wobei das Gemisch (G1) zur Energiegewinnung eingesetzt wird, wobei das Gemisch (G1)
(aa) wenigstens einer weiteren Aufarbeitungsvorrichtung zugeführt wird,
(bb) in dieser verbrannt wird und
(cc) die gemäß (bb) entstehende Verbrennungswärme zur Wasserdampfgewinnung eingesetzt wird, wobei der Wasserdampf als Energieträger zum Betreiben von Destillationskolonnen in dem Verfahren zur Herstellung von Propylenoxid eingesetzt wird.

2. Verfahren nach Anspruch 1, wobei das Gemisch (G1) in (aa) in der wenigstens einen weiteren Aufarbeitungsvorrichtung mit weiterem Sauerstoff versetzt wird.

## Claims

1. A process for preparing propylene oxide which comprises at least the following steps:
(a) propene is reacted with hydrogen peroxide in a solvent in the presence of a zeolite catalyst to give a mixture (M0) comprising at least propylene oxide, solvent, unreacted propene, unreacted hydrogen peroxide and oxygen,
(b) the propylene oxide is separated off from the mixture (M0) so as to give a mixture (M1) comprising unreacted propene and oxygen, and
(c) the mixture (M1) is valorized without, in the course of this valorization, the propene present in the mixture (M1) being returned into the reaction of (a), the mixture (M1) being used for energy recovery in that the mixture (M1)
(aa) is passed to at least one further work-up apparatus,
(bb) is burnt therein and
(cc) the combustion energy produced in (bb) is used to produce steam which is used as an energy transfer medium for operating distillation columns in the process for preparing propylene oxide.

2. The process according to claim 1 wherein the mixture (M1) is admixed in (aa) with further oxygen in the at least one further work-up apparatus.

## Revendications

1. Procédé permettant la fabrication d'oxyde de propylène, lequel comporte au moins les étapes suivantes :
(a) du propène réagit avec du péroxyde d'hydrogène dans un solvant en présence d'un catalyseur zéolitique jusqu'à obtention d'un mélange (G0), le mélange (G0) comprenant au moins de l'oxyde de propylène, du solvant, du propène n'ayant pas réagi, du péroxyde d'hydrogène n'ayant pas réagi et de l'oxygène,
(b) l'oxyde de propylène est séparé du mélange (G0), de façon à obtenir un mélange (G1), lequel contient du propène n'ayant pas réagi et de l'oxygène,
(c) le mélange (G1) étant utilisé, dans le cadre de cette utilisation, le propène contenu dans le mélange (G1) n'étant pas réintroduit dans la réaction selon (a) et le mélange (G1) étant utilisé pour gagner de l'énergie, le mélange (G1)
(aa) étant introduit dans au moins un autre dispositif de traitement,
(bb) étant brûlé dans celui-ci et
(cc) la chaleur résultant de (bb) étant utilisée pour obtenir de la vapeur d'eau, la vapeur d'eau étant utilisée comme source d'énergie permettant l'exploitation de colonnes de distillation dans le procédé permettant la fabrication de l'oxyde de propylène.

2. Procédé selon la revendication 1, le mélange (G1) en (aa) étant mélangé dans le au moins un autre dispositif de traitement avec un autre oxygène.
